# EUROPEAN PATENT APPLICATION

(11) **EP 4 375 929 A1**
(43) Date of publication of application: **29.05.2024**
(21) Application number: 22209751.1
(22) Date of filing: 25.11.2022
(51) Int. Cl.: G06T 7/70

(54) **SYSTEMS AND METHODS FOR REGISTRATION OF COORDINATE SYSTEMS BASED ON 2D X-RAY IMAGING AND AUGMENTED REALITY DEVICE**

(71) Applicant: metamorphosis GmbH, 33184 Altenbeken (DE)
(72) Inventor: Reimer, Stefan, 33100 Paderborn (DE); Lamm, Artur, 33102 Paderborn (DE); Schlenker, Julian, 33098 Paderborn (DE); Vater, Hendrik, 33098 Paderborn (DE); Sieland, Christoph, 32839 Steinheim (DE)
(74) Representative: LKGLOBAL Lorenz & Kopf Patentanwalt Attorney at Law PartG mbB

(57) **Abstract**

Systems and methods are provided for assisting in a musculoskeletal procedure. In general, a first X-ray image is received showing a first object, the first X-ray image being generated by an imaging device. An imaging direction of the first X-ray image is determined based on a 3D representation of the first object, the imaging direction of the first X-ray image being associated with a coordinate system of the first X-ray image. Then, information of the pose of the imaging device is received at which pose the first X-ray image has been generated, wherein the information includes a geometrical aspect of the imaging device and is provided by an augmented-reality device associated with a global coordinate system. Further, a position and orientation of the first object relative to the global coordinate system is determined based on a registration of the coordinate system of the first X-ray image with the global coordinate system.

## Description

### FIELD OF INVENTION

The invention relates to the field of systems and methods in musculoskeletal treatment. In particular, the invention relates to a computer-based assessment of X-ray images and to a visualization of information within a field of view of a physician for assisting the physician in the musculoskeletal treatment. A method in accordance with the invention may be implemented as software program product executable on a processing unit of a system.

### BACKGROUND OF THE INVENTION

In a case in which a bone is fractured, the pieces of the bone may be stabilized by an implant like an intramedullary nail, which may be inserted into the medullary canal of the bone, or a bone plate, which may be affixed to the surface of the bone, as support for the healing of the fracture. The surgical procedure for implanting such implants may be minimally invasive and may require repeated acquisition of X-ray images to enable the surgeon to correctly place the implant. The implant may also be connected to one or more sub-implants, e.g., a screw or a blade.

Steps in the surgical procedure may require determining the locations, shapes, and dimensions of objects (e.g., surgical tools, implants, or bone structures) depicted in an X-ray image. For example, the surgeon may need to determine the length of a blade for implantation by measuring an anatomical structure shown in an X-ray image. However, a reliable measurement of dimensions based on an X-ray image requires calibration.

It is, for example, suggested in the art to utilize a reference body mounted to the X-ray imaging device in order to calibrate acquired X-ray images. Furthermore, it is suggested in the art to use at least one further reference body mounted to an instrumentation. Such a reference body may assist in determining the 3D position of an implant or surgical instrumentation. This allows determining and displaying the relative position of an implant with respect to a bone in a computer-assisted surgery system. Moreover, a reference body may also be required for matching images depicting the femoral head acquired from different directions in order to provide a 3D representation of the femoral head.

A sufficiently correct reduction of a fracture is essential for a satisfactory clinical outcome in any osteosynthesis procedure. Typically, fractures heal satisfactorily only if the reduction was correctly performed. Reductions of long bones in particular are often difficult to evaluate during surgery, especially concerning a correct angle of anteversion. An incorrect angle of anteversion is often noticed only after completed surgery when the patient is able to stand up again. At this stage, an incorrect angle of anteversion causes major discomfort to the patient, even if the fracture itself healed properly. Thus, a sufficiently correct angle of anteversion is essential for a satisfactory clinical outcome, especially for osteosynthesis of the femur and the tibia. Indeed, an incorrect angle of anteversion is one of the major causes for revision surgery.

The prior art proposes different approaches to determine the angle of anteversion. In case of a femur and a cephalomedullary nail, one approach is to determine by hand whether the knee is pointing upwards to the ceiling of the operating room and to judge subjectively whether the screw, which should intersect the nail axis and the center of the femoral head, makes an angle of approximately 10 degrees with the floor of the operating room. Another approach is proposed by US 2015/0265361 A1, where two reference bodies with metallic markers, one in the distal region and one in the proximal region of the femur, and two proximal X-ray images and one distal X-ray image, all depicting the respective reference body, are used.

The main difficulty of determining the angle of anteversion is that the long bones are too long to fit in one X-ray image. Moreover, the geometries required for determining the angle of anteversion are located at the most proximal and most distal parts of the bone, e.g., for the femur, the neck axis and the condyles. Hence, the geometries, which are depicted in separate proximal and distal X-ray images, must be related to each other.

A relation between separate X-ray images may be based on the knowledge of an imaging direction of each of the images. WO 2020/038917 A1 describes a concept for a determination of an imaging direction in which specific anatomical features may be identified by an algorithm in an X-ray image, wherein it may be possible to determine whether the actual imaging direction matches a desired imaging direction based on relative locations of these features in the image. For example, for entry point support for inserting the nail into the bone marrow, an oblique lateral view may be required in order to assess the current position of an opening instrument with respect to the detected bone shaft axis. It may be possible to determine whether the imaging direction is suitable by comparing in the acquired X-ray image the position of, for example, the greater trochanter to the extended bone shaft axis. If these are close, the imaging direction may be considered by the algorithm as acceptable. If these are not close, the imaging direction is not suitable.

### SUMMARY OF THE INVENTION

It may be seen as an object of the invention to provide systems and methods which improves the ability of a physician to evaluate a position and orientation of two objects relative to each other in the field of treatment. It may also be seen as an object of the invention, to provide systems and methods which provide information about a spatial position and orientation of objects as visible in an X-ray image relative to the 3D space of an operation theatre. Based on such information, it may be possible to provide information of two objects relative to each other.

Solutions for these objects are provided by the subject-matter of each of the independent claims. Embodiments are described in the respective dependent claims.

In general, a system is provided with a processing unit, wherein a method is implemented by a computer program product, i.e. a computer software. The computer program product which is executable on the processing unit includes sets of instructions for receiving a first X-ray image showing a first object, wherein the first X-ray image is generated by an imaging device. The first object may be an implant or a tool. Further, the first object may be a bone. In particular, the first object may be a fragment of a bone. It will be understood that the first object may be shown only partially in the X-ray image. The imaging device may be a C-arm based X-ray imaging device.

Further, the computer program product includes sets of instructions for determining an imaging direction of the first X-ray image based on a 3D representation of the first object, the imaging direction of the first X-ray image being associated with a coordinate system of the first X-ray image. Determining an imaging direction of an X-ray image may be understood as eliminating, e.g., three degrees of freedom of an object depicted in the X-ray image, in other words, determining the rotation of the object in the coordinate system of the X-ray image. In the context of this disclosure, a 3D representation may be a model of an object which model may be predetermined or may be generated during the treatment procedure. The model may be a 3D data set of the used implant or tool. In case of a bone, the model may be a statistical model, like a statistical shape model or a statistical appearance model. A bone model may be a 3D representation of a bone that may be used for the detection and/or a pose estimation of the bone. The 3D representation may be the result of a preoperative scan like a computed tomography (CT) scan or a cone beam-based 3D image which may also be acquired intraoperatively, and may be an approximation of a bone with or without preliminary knowledge. The computer program product may further include sets of instructions for generating at least one of the 3D representation of the first object and the 3D representation of the second object. In other words, a "3D representation" may refer to a complete or partial description of a 3D volume or 3D surface, and it may also refer to selected geometric aspects, such as an axis, a plane, a two-dimensional manifold, a sphere or the like. It may be possible to determine complete 3D information including a scaling about the 3D surface or volume of an object, but in many applications, it may be sufficient to determine only selected geometric aspects or ratios of those aspects, e.g. a ratio of width to length.

The computer program product includes set of instructions for receiving information of the pose of the imaging device at which pose the first X-ray image has been generated, wherein the information, which may be a partial information, includes a geometrical aspect of the imaging device and is provided by an augmented-reality device associated with a global coordinate system. Here, the "pose" of the imaging device is intended to mean a spatial position and orientation of the imaging device in the space of the operation theatre. For example, the pose includes distances of the imaging device to, e.g., the floor and the walls of the operation theatre room as well as angles of the radiation source and the radiation detector relative to the floor and the walls of the room. In the context of this disclosure, the global coordinate system relates to the operation theatre room, i.e. to the room in which the imaging device is arranged. The floor and the walls of the room as well as devices in the room are detectable, for example, by sensors of an augmented reality device like a head mounted display so that the augmented reality device may provide information on the basis of which the computer program product of the system may define a global coordinate system. For example, a head mounted display may sense the operation theatre room defining the global coordinate system and may further sense distances and angles of the X-ray radiation detector of the imaging device in relation to that global coordinate system.

An information of a pose may include six translational and rotational parameters. In particular, the information of a pose may consist of less than six parameters.

The computer program product further includes set of instructions for determining a position and orientation of the first object relative to the global coordinate system based on a registration of the coordinate system of the first X-ray image with the global coordinate system.

In a case in which the X-ray image shows the first object together with a tool, the computer program product may further include sets of instructions for eliminating not all degrees of freedom of the position and orientation of the tool in the global coordinate system, for eliminating at least two, e.g. three, four or five, degrees of freedom of the position and orientation of the first object relative to the tool, and for identifying a geometrical aspect in the coordinate system of the first X-ray image. In 3D space, an object like a tool has six degrees of freedom, three translational and three rotational degrees of freedom. A spatial position and orientation of the object may be determined by eliminating the degrees of freedom, i.e. by defining translational values along the three coordinate axes as well as rotational values about the three axes. Here, four or even only two out of the possible six degrees of freedom are eliminated, thus allowing ambiguities. The geometrical aspect in the coordinate system of the first X-ray image may be a 2D manifold, a curve, a point, or a set of points. Even with the mentioned ambiguities, a determination of the position and orientation of the first object relative to the global coordinate system may be based on a relationship between the geometrical aspect of the imaging device and the geometrical aspect in the coordinate system of the X-ray image.

The computer program product may further include sets of instructions for receiving a second X-ray image of a second object, the second X-ray image being generated by the imaging device, and determining an imaging direction of the second X-ray image based on a 3D representation of the second object, the imaging direction of the second X-ray image being associated with a coordinate system of the second X-ray image. The aspects described above with respect to the first object visible in a first X-ray image also apply to a second object in a second image. Likewise, the computer program product may include sets of instructions for receiving information of the pose of the imaging device at which pose the second X-ray image has been generated, wherein the information includes a geometrical aspect of the imaging device and is provided by the augmented-reality device associated with the global coordinate system. In a case in which a second X-ray image is provided, a position and orientation of the second object relative to the first object in the global coordinate system may be determined based on a registration of the coordinate system of the second X-ray image with the global coordinate system.

As mentioned above, the first object may be an implant, a tool, a bone, or just a fragment or part of a bone, wherein the first object may be only partially visible in the X-ray image. The second object may also be one out of those objects (e.g.: another part of the same bone may be fractured or not fractured), leading to several different combinations of first and second objects. For example, the first object may be a bone and the second object may be an implant. Alternatively, the first object may be an implant and the second object may be a tool like a drill or may be a bone screw. Moreover, the first and second objects may both be fragments or parts of one bone. In all cases, it may be of interest to have information about spatial positionings and orientations of the two objects relative to each other.

In a case in which the first object is a first fragment of a fractured bone and the second object is a second fragment of the fractured bone, the computer program product may further include sets of instructions for determining at least one aspect out of the group consisting of a bone length, an angle of anteversion, an angle of version, a centrum-collum-diaphyseal (CCD), ratio of length to width of the bone or of parts of it, and a bone curvature.

The information of position and orientation of an object may be provided to a physician on a screen of a display. For example, a display may be arranged close to the site of treatment so that a physician must only slightly change his/her viewing direction when looking onto the treatment site and onto the display. The information may also be projected onto the X-ray device so that a physician may get the impression to see the same as the X-ray imaging device. Alternatively, an augmented reality device like a head mounted display may be used, wherein the head mounted display is semi-transparent so that the physician may see both the treatment site and the information provided on the display. The computer program product may further include sets of instructions for providing a visualization on the augmented-reality device, wherein the visualization is at least one out of the group consisting of the 3D representation of the first object, the 3D representation of the second object and the tool, and wherein the visualization is shown at the position and orientation as determined in the global coordinate system. It will be understood that the augmented reality device may also show an implant or tool as already inserted into a patient. It will further be understood that another object like a tool, an implant or a bone may be visualized on the augmented reality device, wherein the other object may not be depicted in the X-ray image but only identified by the augmented reality device in the global coordinate system.

Further, the computer program product may include sets of instructions for determining an insertion path for a tool or an implant, and for providing a visualization of the insertion path on the augmented-reality device, wherein the insertion path is shown at the position and orientation as determined in the global coordinate system. For example, the insertion path may be visualized as a volume, a curve or a point on the display of the augmented reality device. Due to the determination of such a path in the global coordinate system, it is possible to virtually hold that path at the 3D position and orientation even if the augmented reality device moves. With such a path in the field of view of the physician, it is facilitated for the physician to manipulate a tool like a scalpel, a drill or an implant so as to follow that path when being inserted into the patient.

Alternatively or additionally, the computer program product may further include sets of instructions for receiving information of a predetermined position and orientation of at least one of the first object and the second object, and to provide a visualization of the information on the augmented-reality device. For example, an anatomically correct position and orientation of two fragments may be visualized in addition to the actual 3D position and orientation thereof. The correct position and orientation may be visualized as outlines of the objects. Such visualization facilitates a repositioning of fragments. Guiding to the correct position may be indicated, for example, by arrows and/or values by the augmented reality device at a suitable position.

Moreover, the computer program product may include sets of instructions for identifying a current status of the treatment of the bone and for providing information suitable for guidance for a next step in the treatment of the bone. As an example, the system may recognize by way of interpreting an X-ray image that an implant has already been inserted into the patient. Following a predetermined sequence of procedural steps, the system may indicate on the display (or by sound) which step should be performed next.

Moreover, the physician and/or the system (by way of the augmented reality device) may detect a movement of the patient or at least of a part of the patient under treatment. In that case, it may firstly be determined whether the movement may affect the treatment. For example, a leg of the patient may move in the direction of a drilling due to a pressure caused by the drilling, e.g., into a bone. Such movement may be assumed as only slightly, if at all, affecting the treatment so that the physician may continue drilling. As another example, a bone may tilt while a screw hole is drilled. Such tilting might require an adjustment of the drilling trajectory. Depending on the actual case, the physician but also the system may suggest generating a new X-ray image. For example, an unintended movement may be detected based on information received from the augmented reality device. Based on the new X-ray image, the system may finally provide guidance for further proceeding in treatment.

A computer program product as described above may be executed on a processing unit of a system. Besides the processing unit, the system may comprise an augmented-reality device and/or an imaging device. In particular, the system may comprise a head mounted display and/or a C-arm based X-ray imaging device. It is envisaged that the imaging device may also comprise separate imaging devices. Otherwise, the imaging device may comprise several combinations of radiation source and radiation detector.

It is noted that a processing unit may be realized by only one processor performing all the steps of the process, or by a group or a plurality of processors, which need not be located at the same place. For example, cloud computing allows a processor to be placed anywhere. For example, a processing unit may be divided into (i) a first sub-processor for processing X-ray image data including a determination of an imaging direction based on the X-ray image, (ii) a second sub-processor for determining a global coordinate system based on information received from an augmented reality device, and (iii) a further processor for controlling a monitor for visualizing results, or a loudspeaker for providing instructions to the user acoustically. One of these or a further processor may also control movements of, for example, a C-arm of an X-ray imaging device.

According to an embodiment, the device may further comprise storage means providing a database for storing, for example, X-ray images. It will be understood, that such storage means may also be provided in a network to which the system may be connected, and that data related to the neural net may be received over that network.

The device may further comprise input means for manually determining or selecting a position or part of an object in the X-ray image, such as a bone outline, for example for measuring a distance in the image. Such input means may be for example a computer keyboard, a computer mouse or a touch screen, to control a pointing device like a cursor on a monitor screen, a controller, hand- or eye-tracking as for a head-mounted display, which may also be included in the device.

A computer program product may preferably be loaded into the random-access memory of a data processor. The data processor or processing unit of a system according to an embodiment may thus be equipped to carry out at least a part of the described process. Further, the invention relates to a computer-readable medium such as a CD-ROM on which the disclosed computer program may be stored. However, the computer program may also be presented over a network like the World Wide Web and can be downloaded into the random-access memory of the data processor from such a network. Furthermore, the computer program may also be executed on a cloud-based processor, with results presented over the network.

As should be clear from the above description, a main aspect of the invention is a processing of X-ray image data and of coordinate systems. The methods described herein are to be understood as methods assisting in a surgical treatment of a patient. Consequently, the method may not include any step of treatment of an animal or human body by surgery, in accordance with an embodiment.

It has to be noted that embodiments are described with reference to different subject-matters. In particular, some embodiments are described with reference to method-type claims (computer program) whereas other embodiments are described with reference to apparatus-type claims (system/device). However, a person skilled in the art will gather from the above and the following description that, unless otherwise specified, any combination of features belonging to one type of subject-matter as well as any combination between features relating to different subject-matters is considered to be disclosed with this application.

### BRIEF DESCRIPTION OF DRAWINGS

Figure 1 shows a flow chart of a method as implemented by a computer program product.
Figure 2 is a schematic illustration of a system according to an embodiment.

### DETAILED DESCRIPTION OF EMBODIMENTS

The aspects defined above and further aspects, features and advantages of the present invention can also be derived from the embodiments / examples to be described hereinafter, but to which the invention is not limited. It will be understood that aspects of the following embodiments / examples may also be combined for advantage. In other words, the following embodiments / examples do not mention all aspects in detail.

### Example 1.1

Given an imaging device that takes an X-ray image in anterior-posterior view at which an augmented-reality device detects a first point of the imaging device in the global coordinate system. Using a model of a bone, the system detects a bone in the X-ray image with respect to an image coordinate system, wherein a second point is identified in the image coordinate system which is known to correspond to the first point in the global coordinate system. The system receives information about the first point from the augmented-reality device and also identifies the image being made in anterior-posterior view based on given knowledge how the detected bone must be oriented in the OR, in order to estimate how the image coordinate system is rotated relative to the global coordinate system, determining the position and orientation of the implant in the global coordinate system.

With the first point in the global coordinate system laying on the second points in the image coordinate system, three translational degrees of freedom are eliminated.

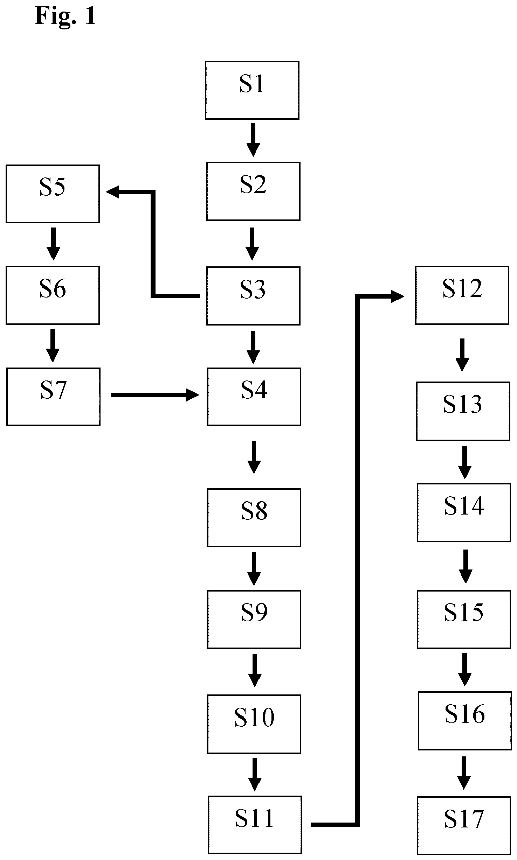

This example may be seen as a method including at least steps S1 to S4 of figure 1.

### Example 2.1

Given a constellation of a femur nail and a drill, identifying an intrinsic axis of the drill.

Given an imaging device that takes a first X-ray image, in which a system detects the pose of the nail in a first image coordinate system, detecting the drill's intrinsic axis in the first image coordinate system (i.e., eliminating four degrees of freedom). An augmented-reality device detects the drill's intrinsic axis in the global coordinate system (i.e., eliminating four degrees of freedom) and detects a first geometrical aspect of the imaging device in the global coordinate system. Given that the first geometrical aspect is known to correspond to a second geometrical aspect in the first image coordinate system, the system can register the first image coordinate system and the global coordinate system by eliminating four degrees of freedom using the detections of the intrinsic axis of the drill in both coordinate systems and by eliminating the remaining two degrees of freedom consisting of the rotation around the intrinsic axis and translation on the intrinsic axis by utilizing the correspondence between the first geometrical aspect and the second geometrical aspect. As a result, the system determines a transformation between the first image coordinate system and the global coordinate system.

This example may be seen as being reflected by the method in figure 1, including, inter alia, steps S5 to S7.

Given the position and orientation of the drill in the global coordinate system at the time of the X-ray image, the augmented-reality device may update the estimation of the drill's intrinsic axis on the basis of detection and/or tracking of aspects of the drill and/or of the power tool. This information can be used to estimate the trajectory of the drill relative to nail or relative to a bone in which the nail is inserted.

Given an estimate of the drill's position and orientation in the global coordinate system, the system may guide the surgeon in adjusting the rotation or position of the drill. It may also provide feedback, e.g., by showing the deviation of the current position or rotation of the drill relative to a target position or rotation. This may also include feedback after drilling started. This may be used with or without new X-ray images, which can be used to recalibrate the estimated position or rotation of the drill.

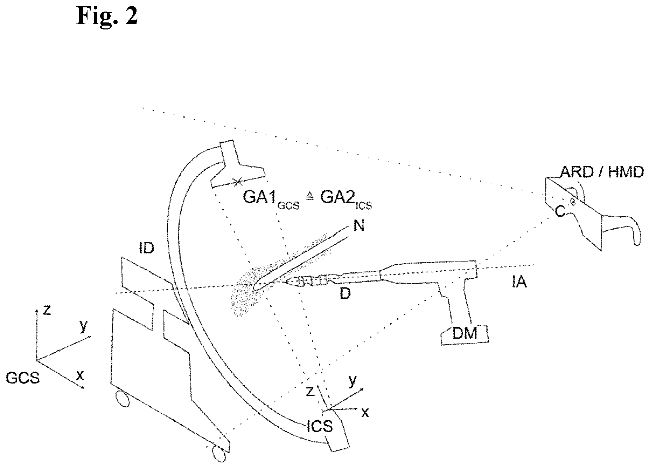

Figure 2 shows the imaging device (ID) which takes an X-ray where a nail (N) is inserted in a bone. The drill (D) may be placed on the bone, which has an intrinsic axis (IA). Part of the drill (D) and the nail (N) are visible in the X-ray image.

The augmented reality device (ARD), in this case a head mounted display (HMD), observes the constellation with a camera (C) which is tracked in the global coordinate system (GCS), the global coordinate system being fixed within the surgery room. The ARD detects the drills intrinsic axis (IA) by detecting a drilling machine (DM), and also detects a point on the image detector of the imaging device (ID) as an example for the first geometrical aspect in the global coordinate system (GA1_GCS).

The system processes the X-ray image in the image coordinate system (ICS) which is fixed relative to the X-ray device (IC) at the time when the image was recorded. The system detects the nail and drills position and orientation in the image coordinate system. Additionally, it identifies a point on the image plane as an example of the second geometrical aspect in the image coordinate system (GA2_ICS).

Then, the fact is utilized that the drill's intrinsic axis (IA) was detected in both coordinate systems. In this case, the relationship between the first and second geometrical aspect is known, namely that both are the same point. The system may register the image coordinate system (ICS) and the global coordinate system (GCS), so that the position and orientation of the nail and drill in the global coordinate system can be detected.

### Example 2.2

Given a constellation of an intramedullary femoral nail and a proximal targeting device implanted in a femur.

Given an imaging device that takes an X-ray image, in which a system is detecting a first, partially incorrect, pose of the nail and a first, partially incorrect, pose of the targeting device in the image coordinate system. An augmented-reality device detects a pose of the targeting device in the global coordinate system and a first geometrical aspect of the imaging device. Identifying a second geometrical aspect in the image coordinate system, utilizing a relationship between the first and second geometrical aspect, the system detects a second pose of the targeting device in the image coordinate system, reducing the estimation error of the first, partially incorrect, pose of the targeting device. Utilizing a spatial relationship between the nail and the targeting device, the system estimates a second pose of the nail, reducing the estimation error of the first, partially incorrect, pose of the nail. Additionally, the system estimates the pose of the nail in the global coordinate system utilizing the spatial relationship between the targeting device and the nail.

Using the estimation of the pose of the nail and targeting device in the global coordinate system and in the image coordinate system, the system may guide the surgeon in adjusting the position of the nail.

Furthermore, the augmented-reality device may estimate the pose of the targeting device after the X-ray image was taken, hence the system may identify a movement of the targeting device. This updated estimation of the targeting device pose in the global coordinate system may be used to guide the surgeon in positioning of the targeting device or nail.

The positioning of the nail may be guided in relation to a previously estimated anatomy, e.g., the proximal femur, wherein a position or orientation of the nail within the bone is proposed to the surgeon.

This proposed nail position in the femur may be shown in the augmented-reality device by visualizing the current position or orientation of the nail in the global coordinate system or relative to the bone. The visualization may also include a blade that should be inserted through the nail after the nail placement would be completed, or a distance between a bone surface and a trajectory based on the current estimate of the nail position or orientation.

### Example 2.3

Given a constellation of a femur nail and a drill, identifying an intrinsic axis of the drill.

Given an imaging device that takes a first X-ray image, in which a system detects the pose of the nail in a first image coordinate system and the system detects the 2D projection of the intrinsic axis of the drill in the image (i.e. eliminating two degrees of freedom). This projection of the intrinsic axis, that is a line in the first X-ray image, directly corresponds to a plane P in 3D of the first image coordinate system. An augmented-reality device detects the drill's intrinsic axis in the global coordinate system (i.e. eliminating four degrees of freedom). Given a first set of two points (e.g. the center of the image intensifier and the center of the X-ray source) on the imaging device in the global coordinate system detected by the augmented-reality device, which corresponds to a second set of two points in the first image coordinate system, wherein the system utilizes the relationship between these two sets of points for a registration of the first image coordinate system and the global coordinate system, wherein the remaining ambiguities of the registration are resolved by the condition, that the intrinsic axis of the drill in the global coordinate system must lie on the plane P in the first image coordinate system. It may be noted that the diameter of the drill can be very small. Instead of a drill, a needle or similar thin tool may be sufficient for the registration of the two coordinate systems.

### Variations of geometrical aspects and their relationships

Examples of the first geometrical aspect are a point on the image intensifier in the global coordinate system, a point on the X-ray source in the global coordinate system, an axis that goes through the center of the X-ray source and through the center of the image intensifier in the global coordinate system, a plane in which that axis lies in the global coordinate system, and a plane in the image intensifier that is parallel to the image in the global coordinate system.

Examples of the second geometrical aspect are a point on the image intensifier in the first image coordinate system, a point on the X-ray source in the first image coordinate system, an axis that goes through the center of the X-ray source and through the center of the image intensifier in the first image coordinate system, a plane in which that axis lies in the first image coordinate system, and a plane in the image intensifier that is parallel to the image in the first image coordinate system.

The relationships between the first and second geometrical aspects are used to eliminate ambiguities or degrees of freedom in order to achieve a registration. The relationships include (but are not limited to) the following options:
- The first geometrical aspect being a set of one or more points and the second geometrical aspect being also a set of one or more points. Each point of the first geometrical aspect should have the same position as one point of the other geometrical aspect. A more specific example of these geometrical aspects is the following. Two points of the first geometrical aspect could be the center of the image plane and the center of the X-ray source of the imaging device in the global coordinate system, which should correspond to two points of the second geometrical aspect in the first image coordinate system after the registration. The two points in either of the geometrical aspect may be known exactly or as an approximation, wherein the registration can also be approximated by minimizing a metric between the geometrical aspects. A metric could be a sum of the L2 norms of the differences between the corresponding points.
- The first geometrical aspect being an axis in the global coordinate system. The second geometrical aspect being an axis in the first image coordinate system. The relationship between these geometrical aspects could be that the two axes should be the same axis after the registration is completed, wherein the first axis lies on the second axis. An example of these axes is the central X-ray beam of the imaging device in the global coordinate system for the first geometrical aspect and the central X-ray beam of the imaging device in the first image coordinate system for the second geometrical aspect. In practice these geometrical aspects may not fit perfectly due to approximations, thus the system can be solved with a metric that describes the distance of the axes in the most relevant spatial region.
- The first geometrical aspect being an axis in the global coordinate system. The second geometrical aspect being an axis in the first image coordinate system. The relationship between these geometrical aspects could be that the two axes should intersect after a successful registration.
- The first geometrical aspect being a point in the global coordinate system. The second geometrical aspect being a plane in the first image coordinate system. The relationship between these geometrical aspects could be that the point should lie on the plane after a successful registration.
- The first geometrical aspect being an axis in the global coordinate system. The second geometrical aspect being a plane in the first image coordinate system. The relationship between these geometrical aspects could be that the axis should lie within the plane after a successful registration.
- The first geometrical aspect being a plane in the global coordinate system. The second geometrical aspect being a plane in the first image coordinate system. The relationship between these geometrical aspects could be that these planes lie on the same plane after a successful registration.

It will be understood that the aforementioned relationships can obviously be utilized on any number of acquired X-ray images and X-ray device positions, not only for the first X-ray image.

### Example 3.1

Given a constellation of a femur bone.

Given an imaging device taking a first X-ray image, which is of the proximal femur bone, wherein a system detects the imaging direction of the bone in the coordinate system of the first image using a model of the bone, wherein an augmented-reality device detects a first geometrical aspect of the imaging device in the global coordinate system.

The imaging device takes a second image, which is of the distal part of the femur bone, wherein the system detects the imaging direction of the bone in the coordinate system of the second image using the model of the bone, wherein the augmented-reality device detects a second geometrical aspect of the imaging device in the global coordinate system. The first and the second geometrical aspect of the imaging device may be overlapping or identical. The system estimates a geometrical aspect of the femur bone utilizing the relationship between the first and second geometrical aspect.

Geometrical aspects may be, but are not limited to, a distance between the proximal and distal end of the femur, a ratio of the width to the length of the femur, a rotation of the distal part relative to the proximal part, an angle of version, an angle of anteversion, a centrum-collum-diaphyseal (CCD) angle, or a bone curvature.

Similar situations may apply also for other long bones.

### Example 3.2

Given a constellation of a femur bone that is fractured, leaving the distal and proximal part of the bone disconnected.

Given an imaging device acquiring a first X-ray image of the proximal femur bone, wherein a system detects the imaging direction of the bone in the coordinate system of the first image using a model of the bone, wherein an augmented-reality device detects a first geometrical aspect of the imaging device in the global coordinate system. The imaging device acquires a second image of the distal part of the femur bone, wherein the system detects the imaging direction of the bone in the coordinate system of the second image using the model of the bone, wherein an augmented-reality device detects a second geometrical aspect of the imaging device in the global coordinate system. The system may determine a geometrical aspect of the femur bone utilizing the relationship between the first and second geometrical aspect.

Geometrical aspects of a bone may be, but are not limited to, a distance, e.g. the distance between the proximal and distal end of the femur, a ratio of distances, e.g. the ratio of the width to the length of the femur, a rotation of the distal part relative to the proximal part, an angle like an angle of version or anteversion, a centrum-collum-diaphyseal (CCD) angle, or a bone curvature.

Geometrical aspects of the imaging device may be a point, a plurality of points or any two dimensional manifold. The first and the second geometrical aspect of the imaging device may be overlapping or identical.

This example could also be extended by acquisition of any number of additional X-ray images and detection of geometrical aspects of the imaging device as described in the example in order to improve the accuracy of the determination of the geometrical aspect of the femur bone.

Similar situations may apply also for other long bones.

### Example 3.3

Using the system of example 3.1 for a healthy long bone to extract a first geometrical aspect, using the system of example 3.2 for a fractured long bone to extract a second geometrical aspect, extracting information to correct the placement of the fractures of the fractured long bone.

### Example 3.4

This example relates to a bone length estimation and to a guidance of a physician.

The estimation of a bone length, as an example of a geometrical aspect of the bone, may be approached based on a sequence of X-ray images. The system uses additional spatial information from an augmented-reality device about an X-ray device at the points in time when the X-ray images were acquired. This additional spatial information may be, e.g., X-ray device positions, X-ray device orientations, a combination of X-ray device positions and orientations, distances between the positions of the X-ray device.

Furthermore, the additional spatial information may include the position and/or orientation about the surgery table or the patient's leg acquired by the augmented-reality device. Based on the additional spatial information, an absolute bone length can be estimated by fitting a statistical model into the X-ray images and measuring the length of the estimated model. The bone length may be generalized as a ratio between two geometrical aspects of the bone, e.g., a ratio between the bone length and the bone width along the femoral neck axis, wherein the absolute values themselves are not required.

Given a reference value of the bone length, e.g. from the healthy bone of the other side of the patient, or derived from other methods of bone length determination, the system may provide guidance to a surgeon for the correct reposition of the fractured bone. The guidance may include instructions for fracture reduction, e.g. on how to stretch, compress or rotate the bone to achieve the reference length. The reference value may be of absolute or relative nature. It may be acquired with the same system on the healthy side, e.g., in case of the femur, the opposite anatomical side.

### Figure 1

Figure 1 is a flow chart illustrating a method in accordance with the disclosure. It will be understood that the steps described are major steps, wherein these major steps might be differentiated or divided into several sub-steps. Furthermore, there might also be sub-steps between these major steps.

In step S1, a first X-ray image is received, the X-ray image showing a first object, wherein the first X-ray image may be generated by an imaging device like an X-ray imaging device.

In step S2, an imaging direction of the first X-ray image is determined based on a 3D representation of the first object, wherein the imaging direction of the first X-ray image is associated with a coordinate system of the first X-ray image.

In step S3, information defining the pose of the imaging device are received at which pose the first X-ray image has been generated, wherein the information may include a geometrical aspect of the imaging device and may be provided by an augmented-reality device associated with a global coordinate system.

In step S4, a position and orientation of the first object relative to the global coordinate system is determined based on a registration of the coordinate system of the first X-ray image with the global coordinate system.

Between steps S3 and S4, the following steps S5, S6 and S7 may be performed in a case in which the first X-ray image further shows a tool.

In step S5, two degrees of freedom of the position and orientation of the tool in the global coordinate system are eliminated, and in step S6, two degrees of freedom of the position and orientation of the first object relative to the tool are eliminated. The order of execution of S5 and S6 does not matter.

In step S7, a geometrical aspect in the coordinate system of the first X-ray image is identified. Based on the results of S5, S6 and S7, a position and orientation of the first object relative to the global coordinate system in step S4 may further be based on a relationship between the geometrical aspect of the imaging device and the geometrical aspect in the coordinate system of the X-ray image.

In some cases, it may be of interest to process a further X-ray image. In those cases, steps S8 to S11 may be performed.

In step S8, a second X-ray image of a second object may be received, wherein the second X-ray image may be generated by an imaging device like an X-ray imaging device.

In step S9, an imaging direction of the second X-ray image may be determined based on a 3D representation of the second object, wherein the imaging direction of the second X-ray image may be associated with a coordinate system of the second X-ray image.

In step S10, information of the pose of the imaging device may be received at which pose the second X-ray image has been generated, wherein the information may include a geometrical aspect of the imaging device and may be provided by the augmented-reality device associated with the global coordinate system.

In step S11, a position and orientation of the second object relative to the first object may be determined in the global coordinate system based on a registration of the coordinate system of the second X-ray image with the global coordinate system.

In step S12, which is an optional step, a 3D representation of the at least one of the objects visible in an X-ray image is generated.

In a case in which the first object is a first fragment of a fractured bone and the second object is a second fragment of the fractured bone, at least one aspect out of the group consisting of a bone length, an angle of anteversion, an angle of version, a centrum-collum-diaphyseal (CCD) angle, and a bone curvature may be determined in optional step S13.

In step S14, which is an optional step, a visualization on the augmented-reality device may be provided, wherein the visualization may be at least one out of the group consisting of the 3D representation of the first object, the 3D representation of the second object and the tool, and wherein the visualization may be shown at the position and orientation as determined in the global coordinate system.

In step S15, which is an optional step, an insertion path for a tool or an implant may be determined, and a visualization of the insertion path may be provided on the augmented-reality device, wherein the insertion path may be shown at the position and orientation as determined in the global coordinate system.

In step S16, which is an optional step, information of a predetermined position and orientation of at least one of the first object and the second object may be received and a visualization of the information may be provided on the augmented-reality device.

In step S17, which is an optional step, a current status of the treatment of the bone may be identified and information suitable for guidance for a next step in the treatment of the bone may be provided.

While embodiments have been illustrated and described in detail in the drawings and the foregoing description, such illustrations and descriptions are to be considered illustrative or exemplary and not restrictive, and the invention is not limited to the disclosed embodiments. The mere fact that certain measures are recited in mutually different dependent claims does not indicate that a combination of these measures can not be used to advantage.

## Claims

1. A computer program product being executable on a processing unit of a system, the computer program product including sets of instructions for
receiving a first X-ray image showing a first object, the first X-ray image being generated by an imaging device,
determining an imaging direction of the first X-ray image based on a 3D representation of the first object, the imaging direction of the first X-ray image being associated with a coordinate system of the first X-ray image,
receiving information of the pose of the imaging device at which pose the first X-ray image has been generated, wherein the information includes a geometrical aspect of the imaging device and is provided by an augmented-reality device associated with a global coordinate system,
determining a position and orientation of the first object relative to the global coordinate system based on a registration of the coordinate system of the first X-ray image with the global coordinate system.

2. The computer program product of claim 1,
wherein the first X-ray image further shows a tool,
the computer program product further including sets of instructions for
eliminating two degrees of freedom of the position and orientation of the tool in the global coordinate system,
eliminating two degrees of freedom of the position and orientation of the first object relative to the tool,
identifying a geometrical aspect in the coordinate system of the first X-ray image,
wherein the determination of the position and orientation of the first object relative to the global coordinate system is further based on a relationship between the geometrical aspect of the imaging device and the geometrical aspect in the coordinate system of the X-ray image.

3. The computer program product of any one of claims 1 and 2, the computer program product further including sets of instructions for
receiving a second X-ray image of a second object, the second X-ray image being generated by the imaging device,
determining an imaging direction of the second X-ray image based on a 3D representation of the second object, the imaging direction of the second X-ray image being associated with a coordinate system of the second X-ray image,
receiving information of the pose of the imaging device at which pose the second X-ray image has been generated, wherein the information includes the or another geometrical aspect of the imaging device and is provided by the augmented-reality device associated with the global coordinate system,
determining a position and orientation of the second object relative to the first object based on a registration of the coordinate system of the second X-ray image with the global coordinate system.

4. The computer program product of claim 3, the computer program product further including sets of instructions for
receiving a priori information of the position and orientation of the second object relative to the first object,
wherein the determination of the position and orientation of the second object relative to the first object is further based on the a priori information of the position and orientation of the second object relative to the first object.

5. The computer program product of any one of claims 1 to 4, the computer program product further including sets of instructions for generating at least one of the 3D representation of the first object and the 3D representation of the second object.

6. The computer program product of any one of claims 3 to 5, wherein the first object is a first fragment of a fractured bone and wherein the second object is a second fragment of the fractured bone, and wherein the computer program product further includes sets of instructions for determining at least one aspect out of the group consisting of a bone length, an angle of anteversion, an angle of version, a centrum-collum-diaphyseal (CCD) angle, and a bone curvature.

7. The computer program product of any one of claims 1 to 6, the computer program product further including sets of instructions for providing a visualization on the augmented-reality device, wherein the visualization is at least one out of the group consisting of the 3D representation of the first object, the 3D representation of the second object and the tool, and wherein the visualization is shown at the position and orientation as determined in the global coordinate system.

8. The computer program product of any one of claims 1 to 7, the computer program product further including sets of instructions for determining an insertion path for a tool or an implant, and for providing a visualization of the insertion path on the augmented-reality device, wherein the insertion path is shown at the position and orientation as determined in the global coordinate system.

9. The computer program product of any one of claims 1 to 8, the computer program product further including sets of instructions for receiving information of a predetermined position and/or orientation of at least one of the first object and the second object, and to provide a visualization of the information on the augmented-reality device.

10. The computer program product of any one of claims 2 to 9, the computer program product further including sets of instructions for receiving information how to reach a predetermined position and/or orientation of the tool or of the imaging device, and to provide the information.

11. The computer program product of any one of claims 1 to 10, the computer program product further including sets of instructions for identifying a current status of the treatment of the bone and for providing information suitable for guidance for a next step in the treatment of the bone.

12. A system comprising a processing unit, wherein the processing unit is configured to execute the computer program according to any one of claims 1 to 11.

13. The system of claim 12, wherein the system further comprises an augmented-reality device.

14. The system of any one of claims 12 to 13, wherein the system further comprises an imaging device.

15. A method assisting in a musculoskeletal procedure, the method comprising steps of
receiving a first X-ray image showing a first object, the first X-ray image being generated by an imaging device,
determining an imaging direction of the first X-ray image based on a 3D representation of the first object, the imaging direction of the first X-ray image being associated with a coordinate system of the first X-ray image,
receiving information of the pose of the imaging device at which pose the first X-ray image has been generated, wherein the information includes a geometrical aspect of the imaging device and is provided by an augmented-reality device associated with a global coordinate system,
determining a position and orientation of the first object relative to the global coordinate system based on a registration of the coordinate system of the first X-ray image with the global coordinate system.
